# EUROPEAN PATENT APPLICATION

(11) **EP 3 581 170 A1**
(43) Date of publication of application: **18.12.2019**
(21) Application number: 18750674.6
(22) Date of filing: 13.02.2018
(51) Int. Cl.: A61K 9/107, A61K 31/4745, A61K 39/39, A61K 47/44, A61K 47/14, A61K 39/00

(54) **NANOEMULSION COMPRISING IMIDAZOQUINOLINE-BASED MATERIAL AND USE THEREOF**

(30) Priority: 13.02.2017 KR 20170019330; 12.02.2018 KR 20180016726
(71) Applicant: Dandi Bioscience Inc, Seoul 05029 (KR)
(72) Inventor: LIM, Yong Taik, Seongnam-si Gyeonggi-do 13599 (KR); KIM, Sun-Young, Daejeon 35265 (KR)
(74) Representative: Dehns
(86) International application number: PCT/KR2018/001891
(87) International publication number: WO 2018/147710

(57) **Abstract**

The present invention relates to a nanoemulsion comprising an oil layer comprising imidazoquinoline-based toll-like receptor 7 or 8 agonist and oil, and a use thereof as an adjuvant and vaccine. According to the present invention, it is possible to provide a vaccine adjuvant in the form of a nanoemulsion that can dissolve an insoluble imidazoquinoline-based material in oil using a dispersion helper and disperse the oil solution easily and reproducibly in a water-soluble manner.

## Description

### [Technical Field]

The present invention relates to a nanoemulsion including an oil layer including an imidazoquinoline-based toll-like receptor 7 or 8 agonist and oil, and a use thereof as an adjuvant or a vaccine.

### [Background Art]

Vaccine adjuvants are immunostimulatory adjuvants which activate immune cells and thus play an important role in vaccines for the prevention/treatment of infectious diseases and anticancer treatment. Aluminum salts (alums) or squalene-based nanoemulsions (MF59, AS03, AF03, and the like), which are immune adjuvants used in conventional vaccines, are effective in inducing improvement in humoral immunity that contributes to antigen reactions, but are fatally disadvantageous in that they have limitations in inducing cellular immunity against viruses and cancer antigens. Thus, nanoemulsion-based adjuvants are restricted to use only as influenza vaccines.

In recent years, there has been a need to develop a vaccine adjuvant capable of effectively inducing both humoral immunity and cellular immunity effects in tuberculosis, HIV, malaria, zoster, cancer vaccines, and the like. To induce cellular immunity, a variety of toll-like receptor ligands, and the like are used. As an example, there is a cervical cancer vaccine commercialized using an AS04 adjuvant preparing by mixing an alum and MPLA, which is a toll-like receptor 4 ligand, in GSK. In addition, AS02 prepared by coating an outer portion of a squalene nanoemulsion with MPLA and saponin-based QS21 has been developed and is in clinical trials. Meanwhile, Korean Patent Registration No. 10-1577955 discloses an adjuvant composition including a composite of a negatively-charged polymer and a toll-like receptor agonist, and a collagen-based material or a gelatin-based material, wherein the adjuvant composition has multistage phase transition properties according to temperature changes. Meanwhile, imidazoquinoline-based drugs (e.g., imiquimod, resiquimod, dactolisib, gardiquimod, sumanirole, and the like), which are known as immune adjuvants for inducing cellular immunity, are toll-like receptor 7 or 8 ligands and have useful properties as vaccine adjuvants.

However, it is very difficult to disperse imidazoquinoline-based materials in an aqueous solution due to molecular structures thereof. In particular, imiqimod (R837) is not dissolved in most organic solvents except for DMSO, and thus has many limitations in being formulated into various preparations. For this reason, imiquimod has been commercialized as a cream-type preparation (Product Name: Aldara® available from 3M) prepared by mixing various surfactants. In addition, imidazoquinoline-based drugs in a salt form (e.g., HCl) may be solubilized in an aqueous solution, but are systemically absorbed to thus cause many toxic effects and side effects, and therefore are not suitable for use in a vaccine adjuvant form.

### [Disclosure]

### [Technical Problem]

The inventors of the present invention had developed an immunostimulatory agent having both immunologic properties of the imidazoquinoline series, which are poorly soluble, and immunologic properties of an oil-based nanoemulsion by dissolving an imidazoquinoline-based toll-like receptor 7 or 8 agonist, which is poorly soluble in an aqueous solution, in oil by using a dispersion helper and preparing the resulting oil mixture into a nanoemulsion form, and had found that the developed agent could be used as an adjuvant or vaccine that enhances not only humoral immunity but also cellular immunity, and thus completed the present invention.

### [Technical Solution]

The present invention provides a nanoemulsion including an oil layer including an imidazoquinoline-based toll-like receptor 7 or 8 agonist and oil.

In addition, the imidazoquinoline-based toll-like receptor 7 or 8 agonist may be dispersed in a nano-sized oil layer.

In addition, an outer portion of the oil layer may be coated with one or more selected from the group consisting of a surfactant and an immunostimulatory agent.

In addition, the imidazoquinoline-based toll-like receptor 7 or 8 agonist may be selected from the group consisting of imiquimod, resiquimod, dactolisib, gardiquimod, sumanirole, motolimod, 3M products such as 3M-052, S-34240, 852, and 854-A, and derivatives thereof.

In addition, the oil may be selected from the group consisting of fish-derived oil, animal-derived oil, vegetable-derived oil, a tocopherol, mineral oil, and castor oil.

In addition, the imidazoquinoline-based toll-like receptor 7 or 8 agonist and the oil may be included in a weight ratio of 0.1:100 to 1:50.

Meanwhile, the nanoemulsion may further include a dispersion helper for assisting a lipophilic interaction between the imidazoquinoline-based toll-like receptor 7 or 8 agonist and the oil.

In addition, the dispersion helper may be selected from the group consisting of myristoleic acid, palmitoleic acid, sapienic acid, oleic acid, elaidic acid, vaccenic acid, linoleic acid, linoelaidic acid, alpha-linoleic acid, arachidonic acid, eicosapentaenoic acid, erucic acid, docosahexaenonic acid, caprylic acid, capric acid, lauric acid, myristic acid, palmitic acid, stearic acid, arachidic acid, behenic acid, lignoceric acid, and cerotic acid.

In addition, the dispersion helper and the imidazoquinoline-based toll-like receptor 7 or 8 agonist may be included in a weight ratio of 0.1:10 to 10:0.1.

In addition, the surfactant may be selected from the group consisting of polyoxyethylene sorbitan ester surfactants (Tween) including polysorbate 20 and polysorbate 80; copolymers including one or more selected from ethylene oxide (EO), propylene oxide (PO), and butylene oxide (BO); octoxynols including Triton X-100 and t-octylphenoxypolyethoxy ethanol; (octylphenoxy)polyethoxy ethanol; phospholipids including phosphatidylcholine(lecithin), phosphatidylethanolaniline, phosphatidylserine, phosphatidylinositol, phosphatidylglycerol, phosphatidic acid, sphingomyelin, and cardiolipin; nonylphenol etoxylates; polyoxyethylene fatty ethers derived from lauryl, cetyl, and oleyl alcohols, including triethyleneglycol monolauryl ether; and sorbitan esters (SPAN) including sorbitan trioleate (Span 85) and sorbitan monolaurate.

In addition, the immunostimulatory agent may be selected from the group consisting of toll-like receptor agonists (TLRs), saponins, antiviral peptides, inflammasome inducers, NOD ligands, cytosolic DNA sensor (CDS) ligands, stimulator of interferon genes (STING) ligands, and cationic lipids.

According to another aspect of the present invention, there is an adjuvant composition including the nanoemulsion.

According to another aspect of the present invention, there is provided a vaccine composition including an adjuvant composition for enhancing both humoral immunity and cellular immunity, and an antigen.

According to another aspect of the present invention, there is provided a method of preparing a nanoemulsion, including: a) preparing an oil solution by dissolving an imidazoquinoline-based toll-like receptor 7 or 8 agonist in oil; b) mixing the oil solution with one or more selected from the group consisting of a surfactant and an immunostimulatory agent; and c) dispersing the mixed solution obtained in process b) in an aqueous solution.

### [Advantageous Effects]

A nanoemulsion including an oil layer including an imidazoquinoline-based toll-like receptor 7 or 8 agonist and oil, according to the present invention, can provide a vaccine adjuvant in an emulsion form which is used in the activation of immune cells that play a vital role in vaccines for the prevention and treatment of infectious diseases and immunotherapy. In addition, an imidazoquinoline-based material is stably dispersed in oil such as squalene using a dispersion helper and the resulting oil layer is finally prepared into an emulsion form, thereby providing a vaccine adjuvant using an immidazoquinoline-based material, which is poorly soluble in most organic solvents and an aqueous solution.

Meanwhile, when an adjuvant in a nanoemulsion form of the present invention is used as a vaccine after binding to an antigen, the adjuvant has an effect of significantly enhancing both humoral immunity and cellular immunity.

In addition, in a nanoemulsion including an oil layer including an imidazoquinoline-based toll-like receptor 7 or 8 agonist and oil, according to the present invention, the imidazoquinoline-based toll-like receptor agonist, which is lipophilic, exhibits controlled release behavior from the oil layer, which is an inner side of the nanoemulsion, to an aqueous solution layer, and thus is slowly released *in vivo,* thereby enhancing cellular immunity and addressing systemic toxicity problems.

### [Description of Drawings]

The above and other objects, features and other advantages of the present invention will be more clearly understood from the following detailed description taken in conjunction with the accompanying drawings, in which:
FIG. 1 is a schematic view illustrating a structure of a nanoemulsion adjuvant composition consisting of an immidazoquinoline-based toll-like receptor agonist, a dispersion helper, oil, a surfactant, and an immunostimulatory agent, according to an embodiment of the present invention;
FIG. 2A illustrates dynamic light scatting (DLS) measurement data obtained by measuring the sizes and size distribution of a nanoemulsion (NE) and a nanoemulsion including an immidazoquinoline-based toll-like receptor agonist(NE-IQ), according to an embodiment of the present invention;
FIG. 2B is a transmission electron microscopy (TEM) image (NE-IQ) showing data obtained by measuring the sizes and size distribution of a nanoemulsion (NE) and a nanoemulsion including an immidazoquinoline-based toll-like receptor agonist(NE-IQ), according to an embodiment of the present invention;
FIG. 3 illustrates the delivery/distribution of NE-IQ according to an embodiment of the present invention into/in immune cells (bone-marrow derived dendritic cells (BMDCs), bone-marrow derived macrophage cells (BMMCs)) (NE-IQ (DID), Lysotracker (FITC)), wherein a shows BMDCs and b shows BMMCs;
FIG. 4 is a set of graphs showing the dependence of expression levels of cytokines related to immune cell maturity after treatment with NE-IQ according to an embodiment of the present invention, on the concentration of imiquimod (R837), wherein a shows BMDCs and b shows BMMCs;
FIG. 5 is a set of graphs showing the dependence of expression rates and viability of surface markers related to immune cell maturity after treatment with NE-IQ according to an embodiment of the present invention, on the concentration of R837, wherein a shows BMDCs and b shows BMMCs;
FIG. 6 is a set of graphs showing effects on the viability of immune cells 24 hours or 48 hours after treatment with NE-IQ according to an embodiment of the present invention, wherein a shows BMDCs and b shows BMMCs;
FIG. 7 illustrates the expression level of IFN-alpha, which is a Type I interferon, after intramuscular injection of NE-IQ;
FIG. 8 illustrates an enzyme-linked immunosorbent assay-IgG titer (3, 6, 9 weeks) of an NE and NE-IQ against an ovalbumin (OVA) antigen, according to an embodiment of the present invention;
FIG. 9 illustrates an enzyme-linked immunosorbent assay-IgG1 titer (3, 6, 9 weeks) of an NE and NE-IQ against an ovalbumin (OVA) antigen, according to an embodiment of the present invention;
FIG. 10 illustrates an enzyme-linked immunosorbent assay-IgG2a titer (3, 6, 9 weeks) of an NE and NE-IQ against an ovalbumin (OVA) antigen, according to an embodiment of the present invention;
FIG. 11 illustrates an enzyme-linked immunosorbent assay-IgG2a/ IgG1 ratio (3, 6, 9 weeks) of an NE and NE-IQ against an ovalbumin (OVA) antigen, according to an embodiment of the present invention;
FIG. 12 illustrates an immunization schedule for measuring activated CTL immune responses after intramuscular injection of NE-IQ;
FIG. 13 illustrates cellular immunity-inducing effects (IFN-gamma secretion by T cell activation) of an NE and NE-IQ against an ovalbumin (OVA) antigen, according to an embodiment of the present invention;
FIG. 14 illustrates the results of measuring cancer cell growth after immunization of B16F10 cancer cell-transplanted mice with NE-IQ;
FIG. 15 illustrates the results of measuring the activation of CD3(+)CD4(+)T cells after immunization of B16F10 cancer cell-transplanted mice with NE-IQ;
FIG. 16 illustrates the results of measuring the activation of CD3(+)CD8(+)T cells after immunization of B16F10 cancer cell-transplanted mice with NE-IQ;
FIG. 17 illustrates the results of measuring the activation of CD4(+)IFN-gamma(+) cells after immunization of B16F10 cancer cell-transplanted mice with NE-IQ;
FIG. 18 illustrates the results of measuring the activation of CD8(+)IFN-gamma(+) cells after immunization of B16F10 cancer cell-transplanted mice with NE-IQ;
FIG. 19 illustrates the results of confirming the inhibition of antigen-specific cancer cell growth after a vaccine including NE-IQ and an ovalbumin antigen was administered to B16F10-OVA cancer cell-transplanted mice;
FIG. 20 illustrates the results of measuring the effect of NE-IQ on cancer cell growth after immunization of EG7-OVA cancer cell-transplanted mice with NE-IQ; and
FIG. 21 illustrates the cellular immunity-inducing effects (IFN-gamma secretion by T cell activation) of NE-IQ-based vaccine adjuvant compositions against an ovalbumin (OVA) antigen, according to an embodiment of the present invention.

### [Best Mode]

Hereinafter, specific embodiments and examples of the present invention will be described in detail with reference to the accompanying drawings in such a way that the present invention may be carried out without difficulty by one of ordinary skill in the art to which the present invention pertains. The present invention may, however, be embodied in many different forms and should not be construed as being limited to the specific embodiments and examples described herein.

Throughout the specification, when an element is referred to as being "on" another element, this includes not only the case where the element is in contact with the other element but also the case where another element is also present between the two elements. Throughout the specification, when a portion is referred to as "including" an element, unless otherwise specifically stated, this indicates that the portion not only does not preclude other elements, but also may further include other elements. As used herein, the terms "about," "substantially," and the like are used to mean a numerical value or a value approximating the numerical value when manufacturing errors and material-allowable errors specific to the mentioned meaning are given, and are used to prevent an unconscientious infringer from improperly using the disclosed details that mention accurate or absolute numerical values to aid in understanding the present invention. "A (performing) step..." or "a step of..." as used throughout the entire specification of the present invention does not mean "a step for...".

In the entire specification, the term such as "combination(s) thereof" included in an expression of the Markush form means a mixture or combination of one or more elements selected from the group consisting of elements described in the expression of the Markush form and includes one or more elements selected from the group consisting of the elements.

The present invention provides a nanoemulsion including an oil layer including an imidazoquinoline-based toll-like receptor 7 or 8 agonist and oil.

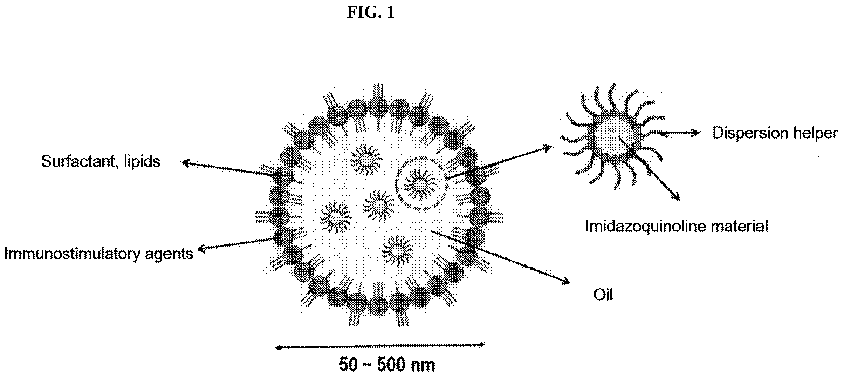

In the nanoemulsion, the imidazoquinoline-based toll-like receptor 7 or 8 agonist may be dispersed in a nano-sized oil layer and an outer portion of the nanoemulsion may be coated with a surfactant, an immunostimulatory agent, or a combination thereof (see FIG. 1).

FIG. 1 is a schematic view of a nanoemulsion of the present invention, wherein the nanoemulsion has a spherical shape and a structure in which the imidazoquinoline-based toll-like receptor 7 or 8 agonist is dispersed in an oil layer, and a surface of the nanoemulsion is coated with a surfactant and/or an immunostimulatory agent. The nanoemulsion may have a size of about 50 nm to about 500 nm, for example, about 50 nm to about 300 nm, about 50 nm to about 100 nm, about 70 nm to about 500 nm, about 100 nm to about 500 nm, or about 100 nm to about 200 nm.

The nanoemulsion may be prepared using the following processes including: a) preparing an oil solution by dissolving an imidazoquinoline-based toll-like receptor 7 or 8 agonist in oil; b) mixing the oil solution with one or more selected from the group consisting of a surfactant and an immunostimulatory agent; and c) dispersing the mixed solution obtained in process b) in an aqueous solution.

In the foregoing description, the imidazoquinoline-based toll-like receptor 7 or 8 agonist may be dissolved in oil along with a dispersion helper for facilitating lipophilic interactions.

The immunostimulatory agent may be used in combination with a surfactant in the nanoemulsion preparation process, or may be additionally attached to the surface of the nanoemulsion after nanoemulsion preparation.

The imidazoquinoline-based toll-like receptor 7 or 8 agonist and the oil may be included in a weight ratio of 0.1:100 to 1:50, for example, 1:20.

The imidazoquinoline-based toll-like receptor 7 or 8 agonist is an immunostimulatory agent and may be selected from the group consisting of imiquimod, resiquimod, dactolisib, gardiquimod, sumanirole, motolimod, 3M products such as 3M-052, S-34240, 852, and 854-A, and derivatives thereof. For example, imiquimod may be used.

The oil may be selected from the group consisting of fish-derived oil, animal-derived oil, vegetable oil-derived oil, a tocopherol, mineral oil, castor oil, and combinations thereof. The fish-derived oil is not particularly limited as long as it is metabolizable oil and includes, for example, cod liver oil, shark liver oil, whale oil, or the like. The shark liver oil contains an unsaturated terpene known as squalene, 2,6,10,15,19,23-hexamethyl-2,6,10,14,18,22-tetracosahexaene, and may also include squalene and saturated analogs for squalene. Squalene and fish oil including squalene are readily available from commercially available sources or may be obtained using a method known in the art. The animal-derived oil may include lard, tallow oil, beef tallow, or the like. The vegetable-derived oil may be oil derived from nuts, seeds, grains, or the like and may include, for example, peanut oil, soybean oil, coconut oil, olive oil, and the like. The tocopherol may be a vitamin E-containing tocopherol. Various tocopherols exist (α, β, γ, δ, ε, or ζ), and α-tocopherol is generally used. For example, DL-α-tocopherol may be used.

The imidazoquinoline-based toll-like receptor 7 or 8 agonist may be solubilized and dispersed in oil using a dispersion helper for facilitating lipophilic interactions with oil, which is poorly soluble. The dispersion helper may, for example, be selected from the group consisting of myristoleic acid, palmitoleic acid, sapienic acid, oleic acid, elaidic acid, vaccenic acid, linoleic acid, linoelaidic acid, alpha-linoleic acid, arachidonic acid, eicosapentaenoic acid, erucic acid, docosahexaenonic acid, caprylic acid, capric acid, lauric acid, myristic acid, palmitic acid, stearic acid, arachidic acid, behenic acid, lignoceric acid, cerotic acid, and combinations thereof. The dispersion helper may be used in a waight ratio of the imidazoquinoline-based toll-like receptor 7 or 8 agonist and the oil in a weight ratio of 0.1:10 to 10:0.1, for example, 1:5 to 5:1.

The surfactant may be coated on an outer portion of the nanoemulsion, enabling the nanoemulsion to be dispersed in an aqueous solution. For example, one selected from polyoxyethylene sorbitan ester surfactants (Tween), particularly polysorbate 20 and polysorbate 80; copolymers such as ethylene oxide (EO), propylene oxide (PO), and/or butylene oxide (BO); octoxynols (e.g., Triton X-100 or t-octylphenoxypolyethoxyethanol; (octylphenoxy)polyethoxy ethanol (IGEPAL CA-630/NP-40); phospholipids (phospholipid component) such as phosphatidylcholine(lecithin) phosphatidylethanolaniline, phosphatidylserine, phosphatidylinositol, phosphatidylglycerol, phosphatidic acid, sphingomyelin, and cardiolipin; nonylphenol etoxylates such as the Tergitol™ NP series; polyoxyethylene fatty ethers derived from lauryl, cetyl, and oleyl alcohols (known as Brij surfactants), such as triethyleneglycol monolauryl ether (Brij 30); and sorbitan esters (generally known as SPAN) such as sorbitan trioleate (Span 85) and sorbitan monolaurate, or a combination of two or more of these surfactants may be used.

For example, as the surfactant, a mixture of these surfactants, for example, a Tween80/Span 85 mixture may be used, or a combination of polyoxyethylene sorbitan ester and octoxynol may be used. Other suitable combinations may include laureth 9, polyoxyethylene sorbitan ester, and/or octoxynol. The surfactant may be used in an amount of 0.001 wt% to 20 wt%, for example, 0.01 wt% to 1 wt%, 0.001 wt% to 0.1 wt%, 0.005 wt% to 0.02 wt%, 0.1 wt% to 20 wt%, 0.1 wt% to 10 wt%, 0.1 wt% to 1 wt%, or about 0.5 wt%, with respect to a total weight of the nanoemulsion.

An additional immunostimulatory agent coated on the outer portion of the nanoemulsion stimulates the proliferation, differentiation and activity of dendritic cells, T cells, and B cells, and accordingly, activated T cells promote differentiation into cytotoxic T cells, which are involved in cellular immunity due to the secretion of cytokines such as interferon-gamma. For example, the additional immunostimulatory agent may include, but is not limited to, a material selected from the group consisting of a toll-like receptor agonist (TLR), a saponin, an antiviral peptide, an inflammasome inducer, an NOD ligand, a cytosolic DNA sensor (CDS) ligand, a stimulator of interferon genes (STING) ligand, cationic ligands, and combinations thereof.

The toll-like receptor agonist may be a natural toll-like receptor agonist or a synthetic toll-like receptor agonist.

The immunostimulatory agent may include a toll-like receptor agonist or a combination of two or more toll-like receptors, and for example, may include CL401 (dual TLR2 and TLR7 agonist) or CL429 (dual TLR2 and NOD2 agonist) alone or a combination thereof, but the present invention is not limited thereto.

The toll-like receptor agonist may be capable of causing a signal transduction response through TLR-1 and may include, for example, one or more materials selected from the group consisting of a tri-acylated lipid peptide (LP); a phenol-soluble modulin; *Mycobacterium tuberculosis* lipid peptides; S-(2,3,-bis(palmitoyloxy)-(2-RS)-propyl)-N-palmitoyl-(R)-Cys-(S)-Ser-(S)-Lys(4)-OH; bacteria lipid peptides derived from *Borrelia burgdorfei*; trihydrochloride (Pam3Cys) lipid peptides that mimic acetylated amino terminals of OspA lipid peptides; and combinations thereof, but the present invention is not limited thereto.

The toll-like receptor agonist may include a TLR-2 agonist and for example, may include, but is not limited to, Pam3Cys-Lip.

The toll-like agonist may include a TLR-3 agonist and for example, may include, but is not limited to, the poly I:C series such as Poly(I:C), poly(ICLC), Poly(IC12U), Ampligen, and the like.

The toll-like agonist may include a TLR-4 agonist, for example, may include, but is not limited to, one or more materials selected from the group consisting of a *Shigella flexneri* outer membrane protein preparation, AGP, CRX-527, MPLA, PHAD, 3D-PHAD, GLA, and combinations thereof.

The toll-like agonist may include a TLR-5 agonist and for example, may include, but is not limited to, flagellin or a fragment thereof.

The toll-like agonist may include a TLR-7 agonist or a TLR-8 agonist and, for example, may include, but is not limited to, imidazoquinoline molecules such as imiquimod, R837, resquimod, or R848; VTX-2337; CRX642; imidazoquinolines covalently bound to phospholipid or phosphono lipid groups; and combinations thereof.

The toll-like agonist may include a TLR-9 agonist and for example, may include, but is not limited to, an immunostimulatory oligonucleotide.

The immunostimulatory oligonucleotide may include one or more CpG motifs, but the present invention is not limited thereto.

The saponin may be selected from the group consisting of QS21, Quil A, QS7, QS17, β-escin, digitonin, and combinations thereof, but the present invention is not limited thereto.

The antiviral peptide may include KLK, but the present invention is not limited thereto.

The inflammasome inducer may be trehalose-6,6-dibehenate (TDB), but the present invention is not limited thereto.

The NOD ligand may be, but is not limited to, M-TriLYS (NOD2 agonist-synthetic muramyltripeptide) or an NOD2 agonist (N-glycolylated muramyldipeptide).

The CDS ligand may be, but is not limited to, Poly(dA:dT).

The STING ligand may be, but is not limited to, cGAMP, di-AMP, or di-GMP.

The immunostimulatory agent may include, but is not limited to, one or more materials selected from the group consisting of Pam3Cys-Lip, Poly(l:C), CRX-527, monophosphoryl lipid A (MPLA), flagellin, imiquimod, resquimod, CpG, QS21, M-TriLys (MurNAc-Ala-D-isoGln-Lys), trehalose-6,6-dibehenate (TDB), 8837, Poly(dA:dT), cGAMP, and combinations thereof.

The cationic lipid may include, but is not limited to, a material selected from the group consisting of 3β-[N-(N',N'-dimethylaminoethane)-carbamoyl]cholesterol hydrochloride (DC-cholesterol), dimethyldioctadecylammonium (DDA), 1,2-dioleoyl-3-trimethylammonium-propane (DOTAP), 1,2-di-O-octadecenyl-3-trimethylammonium propane (DOTMA), 1,2-dimyristoleoyl-sn-glycero-3-ethylphosphocholine (EPC), Nl-[2-((1S)-1-[(3-aminopropyl)amino]-4-[di(3-aminopropyl)amino]butylcarb oxamido)ethyl]-3,4-di[oleyloxy]-benzamide (MVL5), lipids 1,2-dioleoyl-3-dimethylammonium-propane (DODAP), and combinations thereof.

The present invention also provides an adjuvant composition including the nanoemulsion according to the present invention, and a vaccine composition including the adjuvant composition according to the present invention and an antigen.

The antigen may be selected from the group consisting of proteins, recombinant proteins, glycoproteins, genes, peptides, polysaccharides, lipopolysaccharides, polynucleotides, cells, viruses, and combinations thereof. The protein may include, for example, ovalbumin, a peptide, a recombinant protein, a subunit, and a split protein antigen. The gene may include a DNA or mRNA antigen, and the peptide may include a cancer cell- or virus-specific peptide antigen. The cell may include, for example, a dendritic cell, a cancer cell, and a cancer cell-derived lysate, and the virus may include, for example, influenza and highly pathogenic influenza.

The adjuvant composition or the vaccine composition may include, in addition to a pharmaceutically effective amount of the nanoemulsion and/or the antigen, one or more pharmaceutically acceptable carriers or diluents.

As used herein, the term "pharmaceutically effective amount" refers to an amount sufficient for a physiologically active ingredient to be administered to an animal or a human to exhibit desired physiological or pharmacological activity. However, the pharmaceutically effective amount may appropriately vary depending on the severity of symptoms, the age, body weight, heath condition, and gender of a patient, administration route, treatment period, and the like.

In addition, as used herein, the term "pharmaceutically acceptable" means physiologically acceptable and when administered to a human, generally not causing allergic responses such as gastrointestinal disorders and dizziness or responses similar thereto. Examples of suitable carriers, excipients and diluents include lactose, dextrose, sucrose, sorbitol, mannitol, xylitol, erythritol, maltitol, starch, acacia gum, alginates, gelatin, calcium phosphate, calcium silicate, cellulose, methyl cellulose, polyvinylpyrrolidone, water, methylhydroxy benzoate, propylhydroxy benzoate, talc, magnesium stearate, mineral oil, and the like. In addition, the composition may further include a filler, an anti-coagulant, a lubricant, a wetting agent, a flavoring, an emulsifier, a preservative, or the like.

In addition, the compositions of the present invention may be formulated using methods known in the art so as to provide rapid, sustained or delayed release of the active ingredient after administration to a mammal, and may be formulated into various forms for oral or parenteral administration. Preparations may be in the form of powders, granules, tablets, emulsions, syrups, aerosols, soft or hard gelatin capsules, sterile injectable solutions, or sterile powders.

The composition according to the present invention may be administered via various routes including oral, transdermal, subcutaneous, intravenous, or intramuscular administration, and the dose of the active ingredient may be appropriately selected depending on various factors such as administration route, the age, gender, and body weight of a patient, the severity of a patient, and the like. In addition, the composition of the present invention may be administered in combination with a known compound capable of synergizing the desired effect.

Advantages and features of the present invention and methods of achieving them will become apparent with reference to the embodiments described in detail below. Hereinafter, the present invention will be described in detail with reference to the following examples. However, these examples are provided for illustrative purposes only and are not intended to limit the scope of the present invention.

### [Mode of the Invention]

### Example 1. Preparation of Squalene Solution Including Imiquimod (R837)

20 mg of imiquimod (TCI, Tokyo, Japan) was dissolved in 100 mg of oleic acid (Sigma Aldrich, USA) with a bath sonicator (Branson Bransonic® MH Mechanical Bath 5800, Emerson, St. Louis MO) at room temperature until the solution became transparent. Thereafter, 1 mL of squalene (5% v/v, Sigma Aldrich, USA) was added thereto and stirred using a blender (Vortex-Genie 2, Scientific Industries, USA) to obtain a uniform squalene solution.

### Example 2. Preparation and Characterization of Squalene Nanoemulsion (NE-IQ) Including Imiquimod

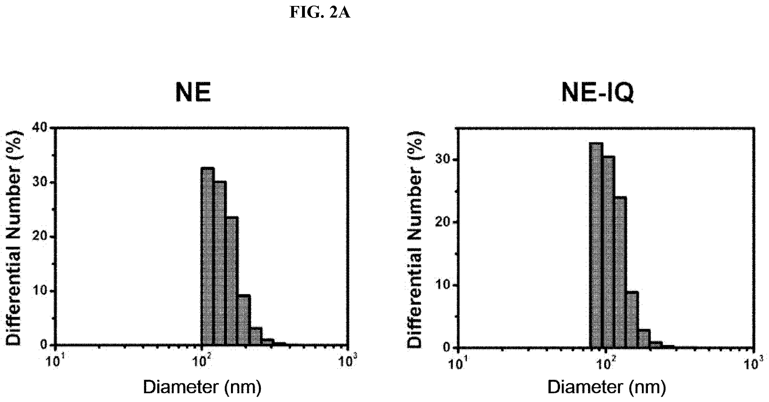

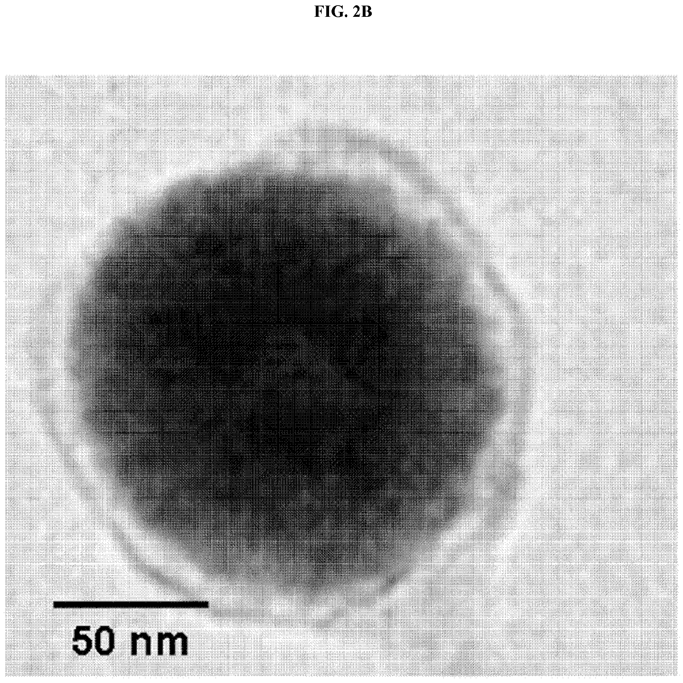

The imiquimod-containing squalene prepared according to Example 1 (5% v/v, Sigma Aldrich, USA), Tween 80 (0.5% v/v, Sigma Aldrich, USA), and Span 85 (0.5% v/v, Sigma Aldrich, USA) were dissolved in 2 mL of phosphate buffer (PBS, 0.0067M PO₄), and then dispersed completely in a phosphate buffer solution (PBS) for 1 minute using an ultrasonic disperser (Tip sonicator). Thereafter, the mixture was stirred for about 2 hours using a tube revolver, and then stored in a refrigerator at 4 °C until use. The size of the emulsion was analyzed by dynamic light scattering (DLS, Otsuka, Japan). As a result of dynamic light scattering (DLS) measurement, it was confirmed that diameters were 106.16 ± 5.4 and 148.54 ± 18.5 nm (see FIG. 2 and Table 1).

**[Table 1]**

| **Formulation** | **Mean diameter ±SD (nm)** |
|---|---|
| **NE** | **106.16±5.4** |
| **NE-IQ** | **148.54± 18.5** |

### Example 3. Introduction of NE-IQ into Immune Cells and Localization thereof in the Cells

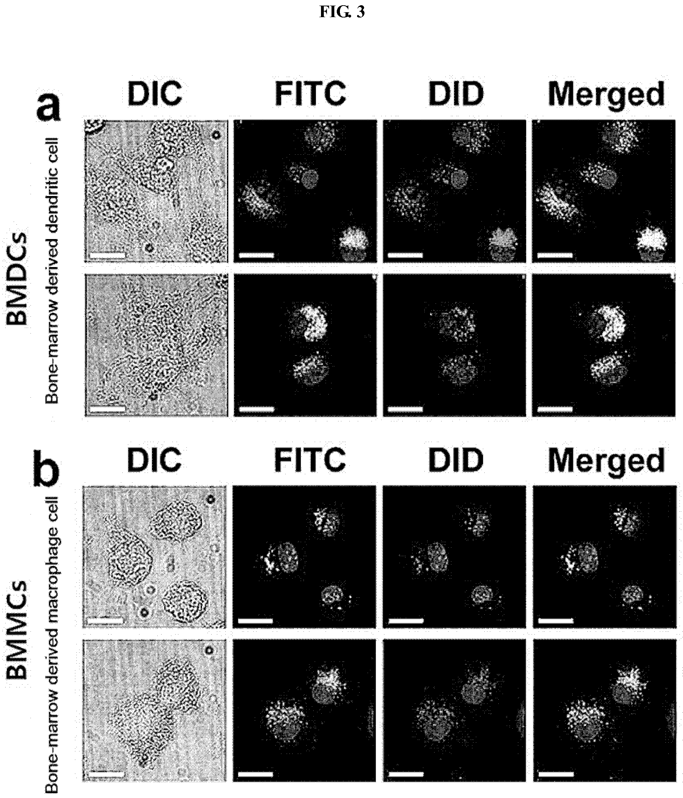

Bone marrow-derived dendritic cells (BMDCs) and bone marrow-derived macrophage cells (BMMCs) were treated with squalene including imiquimod (R837) for 24 hours, and then evaluated using a fluorescence microscope. As shown in the fluorescence image of FIG. 3, lysotracker (green) and DID (red) were found to co-localize at the same position in the cells. From these results, it was confirmed that the squalene nanoemulsion including imiquimod (NE-IQ) were located in endosomes and lysosomes.

### Example 4. Evaluation of Cell Activation Effect of NE-IQ on BMDCs and BMMCs

The effect of NE-IQ on the activation of bone marrow-derived dendritic cells (BMDCs) and bone marrow-derived macrophages (BMMCs) was examined through secretion amounts of proinflammatory cytokines (TNF-α, IL-6, IL-12, IL-2) using ELISA.

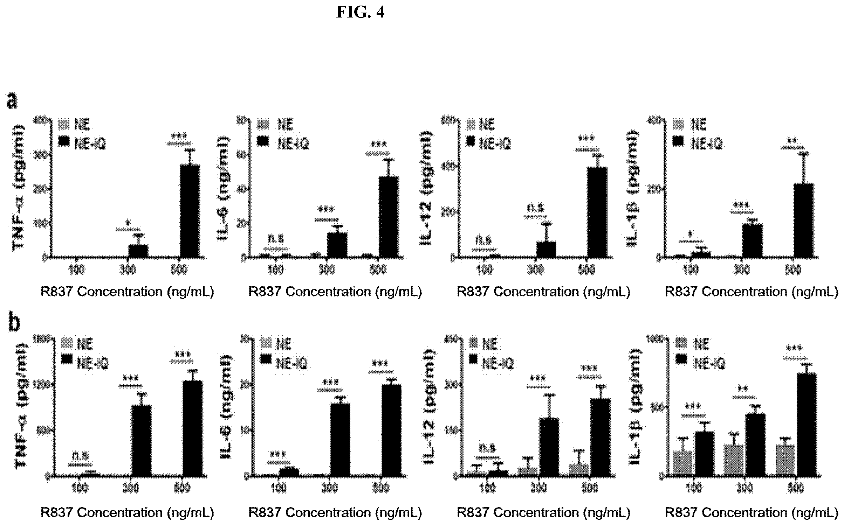

In FIG. 4, when treated with NE-IQ, it was confirmed that the secretion of IL-6 and IL-12 that induce a Th1 response was increased. It was also confirmed that the secretion amounts of TNF-α, which induces apoptosis and inhibition of tumorigenesis, and the inflammatory cytokine IL-1β were also significantly increased compared to a case in which imiquimod (R837) was not included.

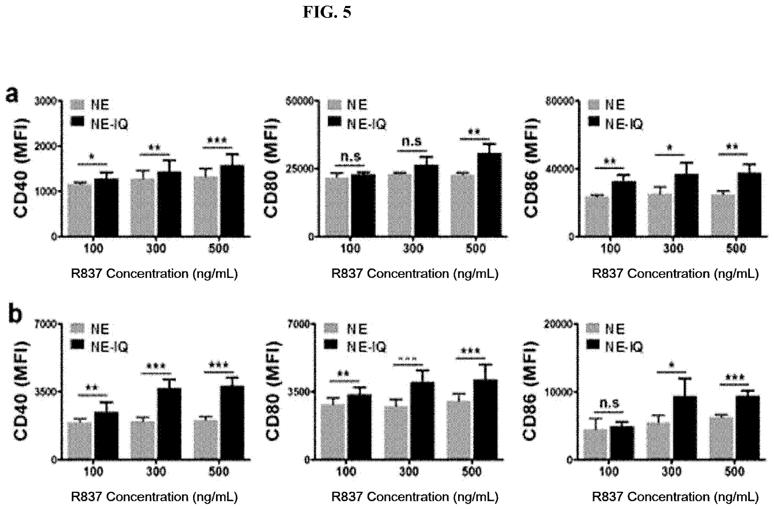

In addition, as a result of labeling CD40, 80, and 86 expressed as BMDCs and BMMCs treated with the sample became mature, it was confirmed that all expression levels in the case of NE-IQ treatment were increased (see FIG. 5). From these results, it was confirmed that the maturation of BMDCs and BMMCs was induced through imiquimod stimulation.

### Example 5. Evaluation of Cell Proliferation Effect of NE-IQ on BMDCs and BMMCs

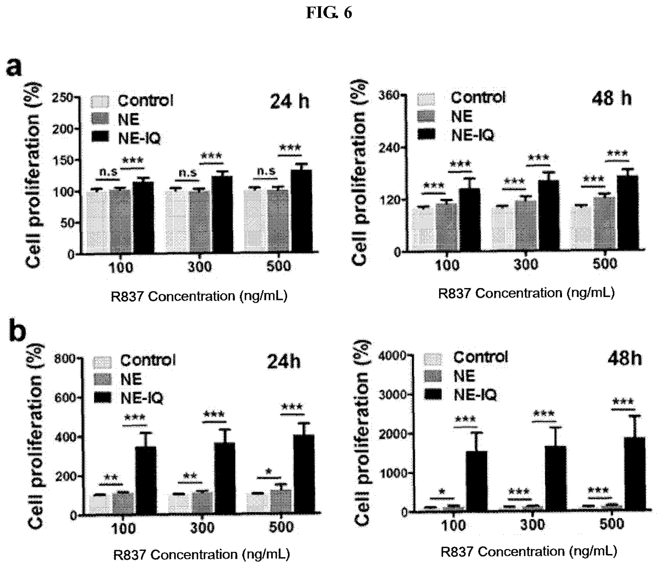

Bone marrow-derived dendritic cells (BMDCs) and bone marrow-derived macrophages (BMMCs) were treated with NE-IQ, and then cell proliferation rates thereof were evaluated. For the evaluation of proliferation rates of FIG. 6, an MTS Assay kit (cell proliferation) assay for measuring mitochondrial activity was used. It was confirmed that the proliferation rates of the two types of immune cells were significantly increased 10-fold or higher when treated with NE-IQ at 24h and 48h compared to the case in which imiquimod was not included.

### Example 6. Evaluation of IFN-α Production Ability of NE-IQ

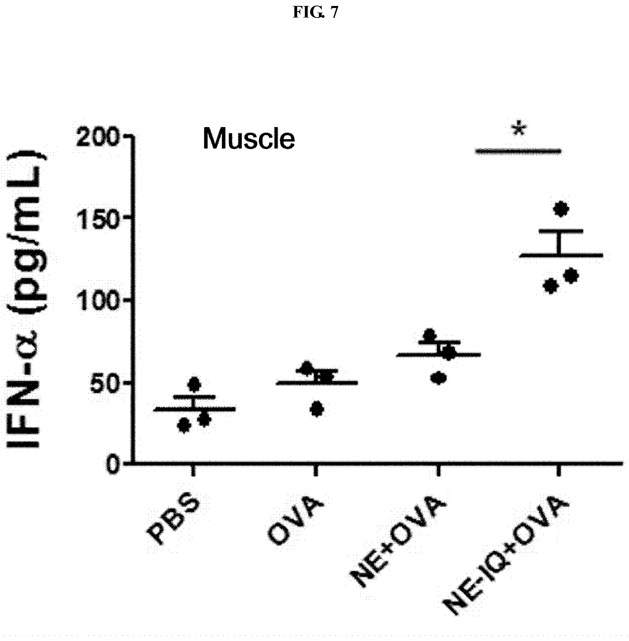

C57BL/6 (female, 5-6 week old, Orient) mice were used in mouse experiments, and 10 µg of ovalbumin (OVA) and 25 µg of imiquimod (R837), which were doses per mouse, were dissolved in 50 µL of NE-IQ. The secretion amount of IFN-α at a muscle site and a lymph node site was analyzed by ELISA 24 hours after the vaccine was injected into each mouse. It was confirmed that the secretion amounts of cytokines were increased in the experimental group treated with NE-IQ compared to other controls (controls using an NE and an alum adjuvant (see FIG. 7). Imiquimod (R837) is known to increase the secretion of interferon (IFN) cytokines through stimulation of toll-like receptor 7.

### Example 7. Confirmation of Antibody-Forming Ability and Cell-Mediated T Cell Activation Effect of Vaccine Including NE-IQ Adjuvant

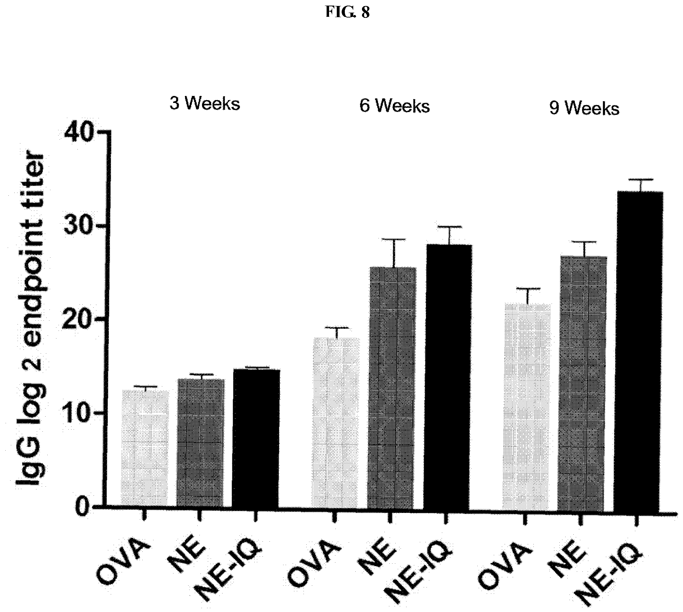

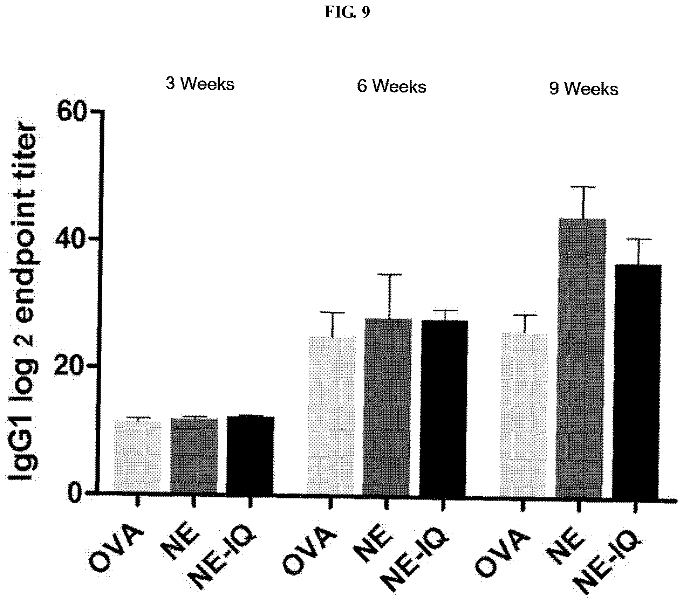

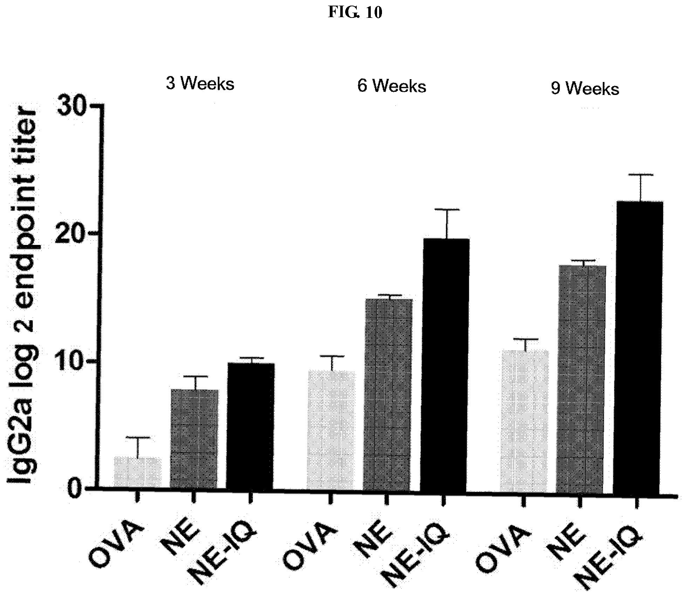

In mouse experiments, C57BL/6 (female, 5-6 week old, Orient) mice were used, and 100 µg of ovalbumin (OVA) and 45.5 µg of imiquimod (R837), which were doses per mouse, were dissolved in 100 µL of NE-IQ prepared in the same manner as in Example 2. An increase in humoral immunity as the vaccine was injected into each mouse was examined by ELISA. For the humoral immunity, a total of three vaccinations were carried out, and after the vaccinations were completed, orbital blood collection was performed on the mice at intervals of 3 weeks and the amounts of produced IgG1 and IgG2a, which are immunoglobulin IgG subtypes, were compared with those of a control (FIG. 8: IgG production amount, FIG. 9: IgG1 production amount, FIG. 10: IgG2a production amount).

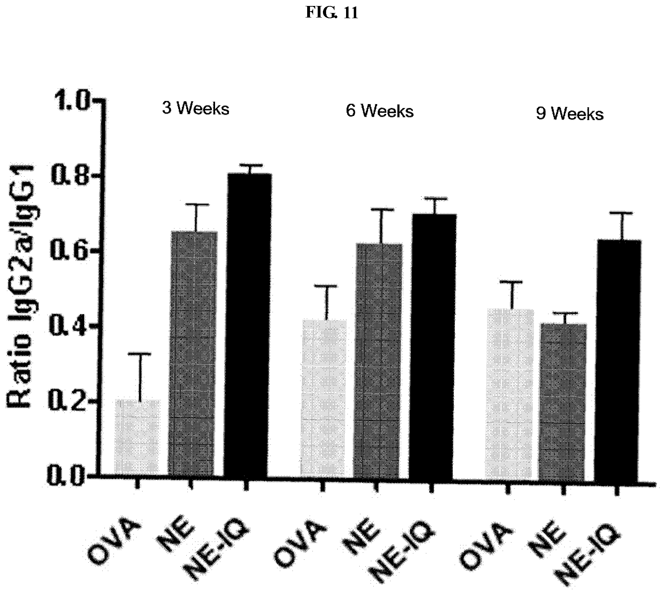

As a result, it was confirmed that the antibody production was increased in all cases of vaccination, and in particular, it was observed that the effect is most significantly exhibited in the vaccine using, as an adjuvant, NE-IQ including imiquimod. In particular, it was confirmed that the experimental group using NE-IQ as an adjuvant exhibited the highest IgG2a/IgG1 value, which exhibits the degree of improvement in cell immunity (see FIG. 11). This is because of the role of imiquimod loaded inside the NE.

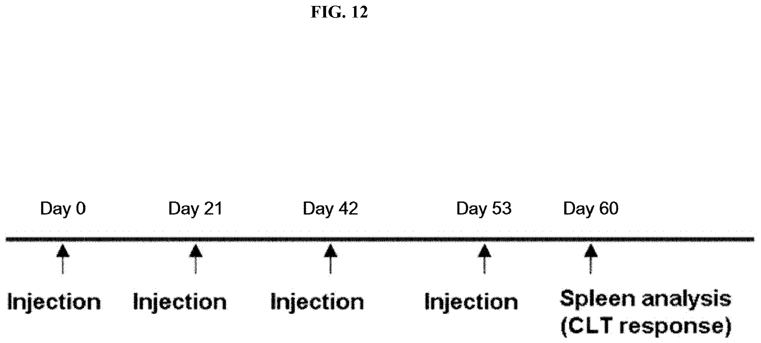

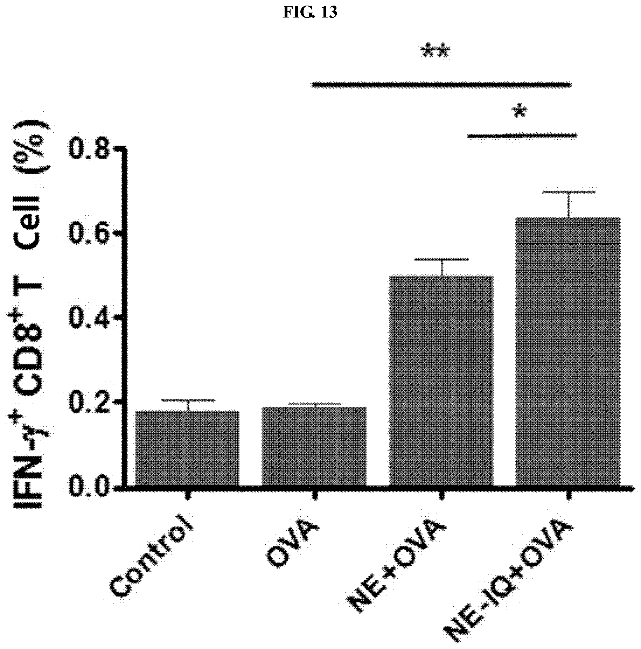

To examine the effect of inducing cellular immunity, immunization was carried out according to a schedule as shown in FIG. 12. Seven days after last injection, spleens were extracted from the mice and single cells were obtained, followed by washing using PBS and centrifugation (1500 rpm, 5 minutes). The single cells were reactivated using OVA peptides, and then as a result of identifying intracellular IFN-gamma in CD8, which is a surface molecule of a T cell, it was confirmed that the activation of CD8+ T cells that secrete IFN-gamma was promoted in the group immunized with NE-IQ compared to a control (see FIG. 13).

### Example 8. Evaluation of Effect of NE-IQ on Inhibiting Cancer Cell Growth

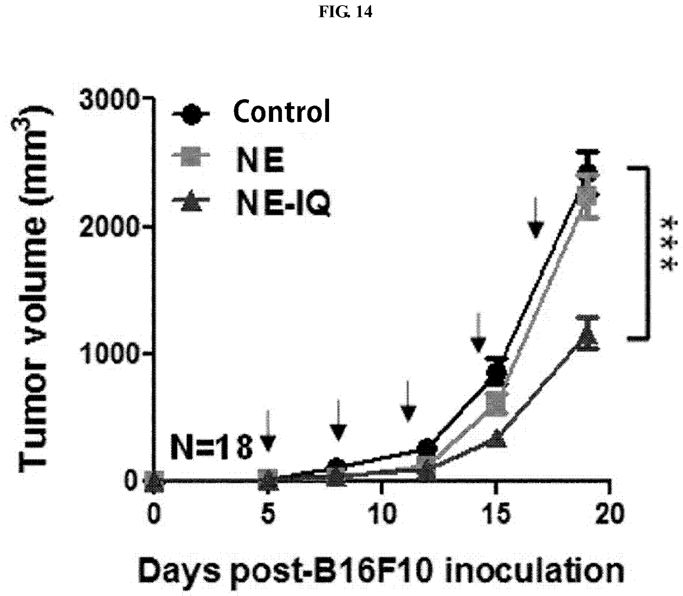

NE-IQ was administered to a C57BL/6 mouse melanoma model via intratumoral injection, and then tumor healing ability thereof was examined. 1 x 10⁵ melanoma cells (B16F10) were injected subcutaneously into the femoral region on the right side of each of 5 female mice aged 6 weeks, and after 5 days, 100 µl of NE-IQ was injected five times into each mouse at intervals of 3 days, and PBS was injected into a control. Thereafter, a tumor volume was measured two to three times per week for 19 days after cancer cell administration. As a result, it was observed that the proliferation of tumor cells was significantly inhibited in the case of NE-IQ administration after cancer cell injection, compared to a control (see FIG. 14).

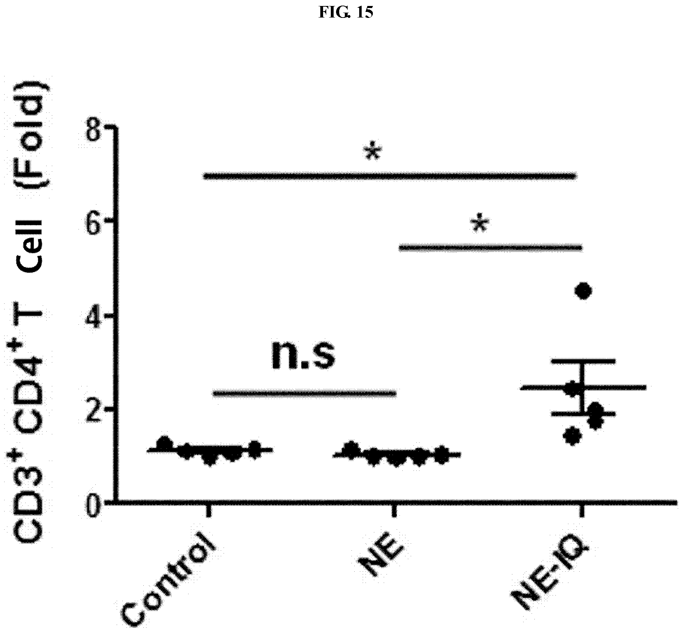

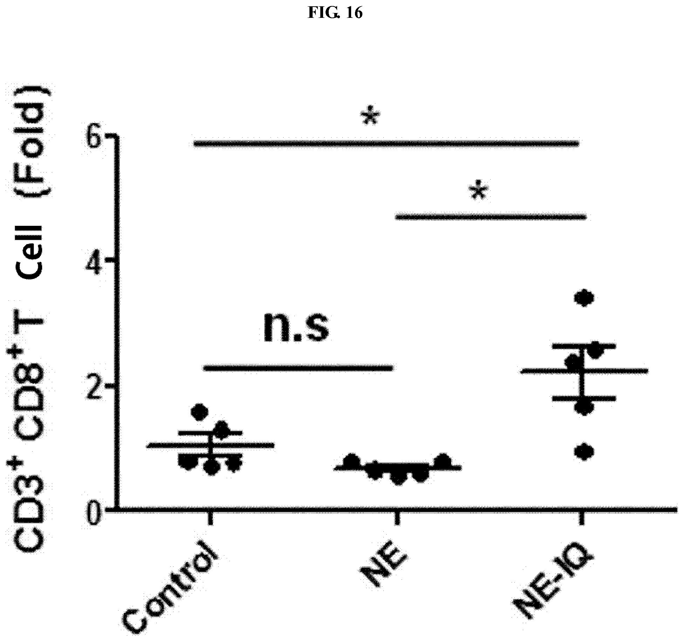

3 days after the last injection, as a result of examining the penetration rate of T cells in the cancer cell area, it was confirmed that the penetration rates of CD4 T cells and CD8 T cells were increased in the case of NE-IQ administration compared to the control (see FIGS. 15 and 16).

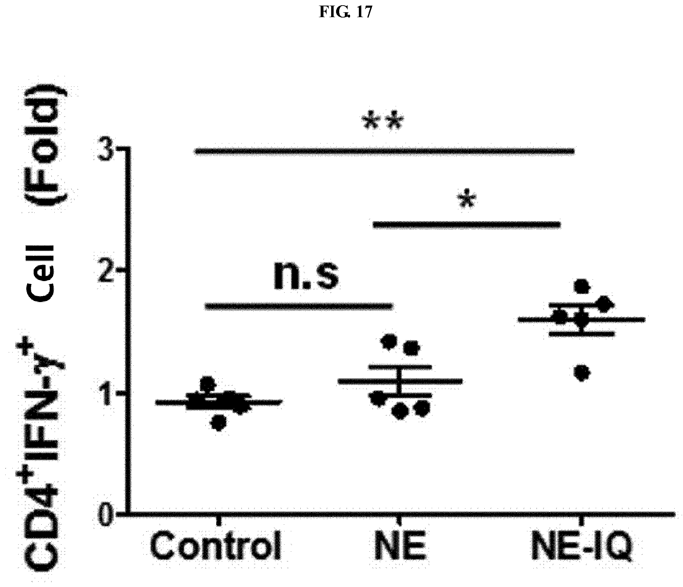

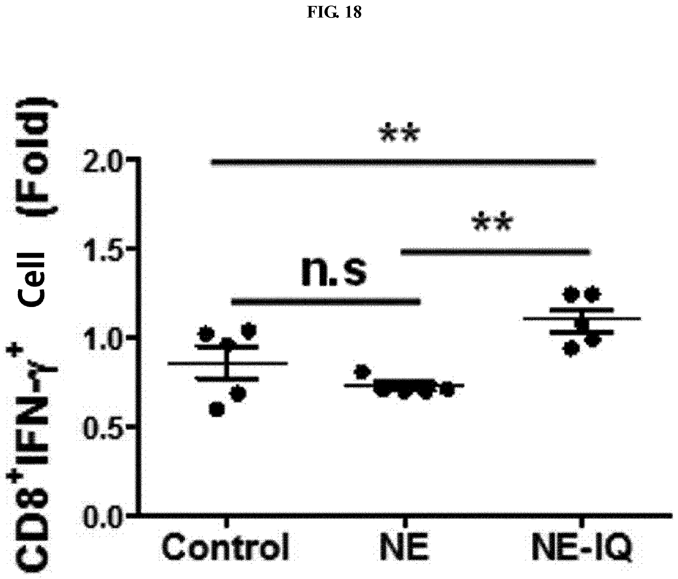

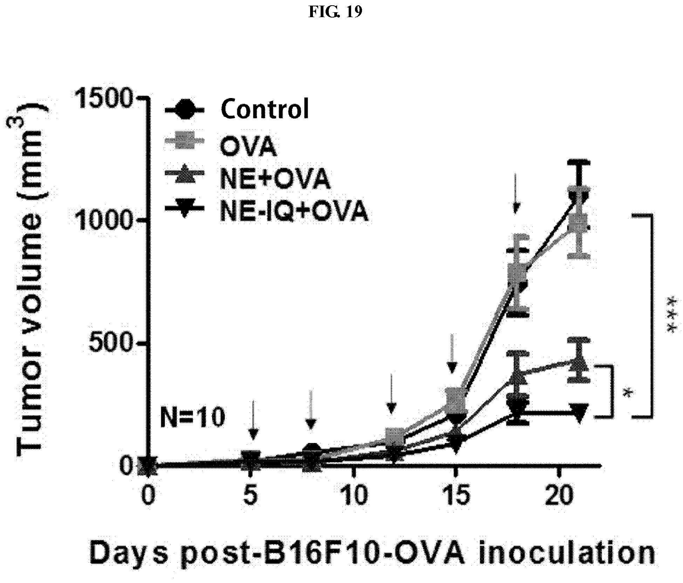

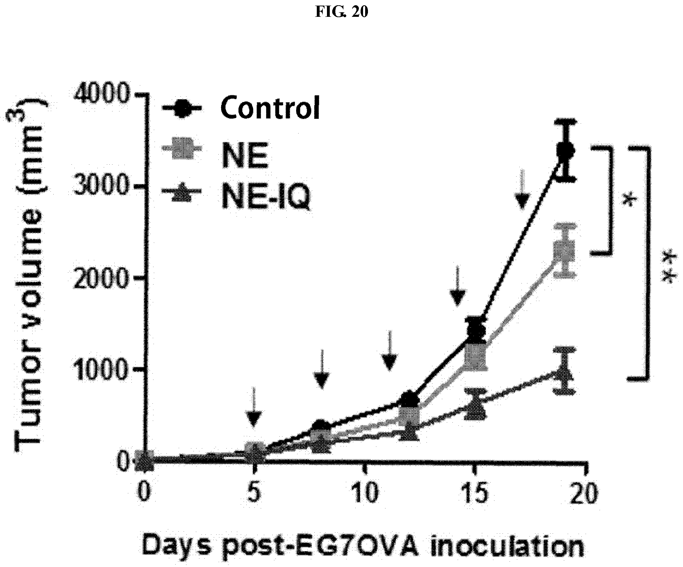

This plays a role in suppressing cancer by increasing the number of T cells that can kill cancer cells. Seven days after the last injection, spleens were extracted from the mice, and single cells were obtained, washed with PBS, and centrifuged (1,500 rpm, 5 minutes). The single cells were reactivated using a B16F10 lysate, and then as a result of identifying intracellular IFN-gamma in CD8 and CD4, which are surface molecules of T cells, it was confirmed that the activation of CD8+T cells that secrete IFN-gamma was promoted in the group administered NE-IQ compared to the control (FIGS. 17 and 18). In addition, a vaccine consisting of NE-IQ and an ovalbumin antigen was administered to a melanoma model (B16F10-OVA) expressing the peptide of ovalbumin (OVA) using the same method as illustrated in FIG. 12, and then antigen-specific inhibition of cancer cell proliferation was confirmed (see FIG. 19). In addition, the effect of NE-IQ on inhibiting cancer cell proliferation was examined using another tumor model. NE-IQ was administered to a lymphoma model (EG7-OVA) expressing a C57BL/6 mouse OVA antigen via intratumoral injection, and then tumor healing ability thereof was examined. 1 x 10⁵ lymphoma cells (EG7-OVA) were injected subcutaneously into the femoral region on the right side of each of 5 female mice aged 6 weeks, and after 5 days, 100 µl of NE-IQ was injected five times into each mouse at intervals of 3 days, and PBS was injected into a control. Thereafter, a tumor volume was measured two to three times per week for 19 days after cancer cell administration. As a result, it was observed that, after cancer cell injection, the proliferation of tumor cells was significantly inhibited in the case of NE-IQ administration compared to a control (see FIG. 20).

### Example 9. Preparation of Nanoemulsion Including One or More Additional Immunostimulatory Agents in NE-IQ

Nanoemulsions including one or more additional immunostimulatory agents such as MPLA, DDA, QS21, and the like in a nanoemulsion (NE-IQ) were prepared according to the compositions shown in Table 2 below.

**[Table 2]**

| Nanoemulsion | Component |
|---|---|
| **NE-IQ-0** | **NE-IQ** |
| **NE-IQ-1** | **NE-IQ + DDA + MPLA** |
| **NE-IQ-2** | **NE-IQ + MPLA + QS21** |
| **NE-IQ-3** | **NE-IQ + Pam3Cys-Lip + QS21** |
| **NE-IQ-4** | **NE-IQ + DDA + STING (cyclic DNA)** |
| **NE-IQ-5** | **NE-IQ + DDA + Poly(l:C)** |
| **NE-IQ-6** | **NE-IQ + DDA + CL401** |
| **NE-IQ-7** | **NE-IQ + DDA + CL429** |
| **NE-IQ-8** | **NE-IQ + DDA + CpG** |

### Example 9-1. Preparation of NE-IQ-1 Adjuvant: NE-IQ + DDA + MPLA

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), cholesterol (0.25 mg/mL, Sigma Aldrich, USA), MPLA (monophosphoryl lipid a, 0.2 mg/mL, Avanti Polar Lipids, USA) were dissolved in 1 mL of ethanol. The solution was transferred to a round flask and the ethanol was completely evaporated using a rotary evaporator to form a lipid thin-film type. Then, a nanoemulsion including imiquimod (R837) was prepared using the same preparation method as that used in Examples 1 and 2, except that mineral oil (2.5% v/v, Sigma Aldrich, USA) was used instead of squalene. 2 mL of a mineral oil-containing solution and a Tween (1% v/v)-containing phosphate buffer solution (PBS, 0.0067M PO₄) was added thereto, and then the lipid film was dispersed in the solution using a stirrer at 60 °C and 600 rpm for 30 minutes. The dispersion solution was transferred to a 4 mL vial, and then a tip sonicator was used for 1 minute to completely disperse the lipid film in the PBS. Thereafter, the resulting solution was stirred using a tube revolver for about 2 hours and then stored in a refrigerator at 4 °C until use.

### Example 9-2. Preparation of NE-IQ-2 Adjuvant: NE-IQ + MPLA + QS21

DOPC (1,2-dioleoyl-sn-glycero-3-phosphocholine, 1 mg/mL, Sigma Aldrich, USA), MPLA [0.2 mg/mL, Avanti Polar Lipids, USA]], a saponin (QS21, 0.2 mg/mL), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Thereafter, an NE-IQ (MPLA + QS21) adjuvant was prepared in the same manner as in Example 2, except that resquimod (R848) and linoleic acid (Sigma Aldrich, USA) were used instead of imiquimod (R837) and oleic acid used in Example 1, thereby preparing a squalene oil solution in which resquimod was dispersed.

### Example 9-3: Preparation of NE-IQ-3 Adjuvant: NE-IQ + Pam3Cys-Lip + QS21

DOPC (1,2-dioleoyl-sn-glycero-3-phosphocholine, 1 mg/mL, Sigma Aldrich, USA), Pam3Cys-Lip (0.2 mg/mL, Invivo Gen, USA), and QS21 (0.2 mg/mL) were dissolved in 1 mL of ethanol. The solution was transferred to a round flask and the ethanol was completely evaporated using a rotary evaporator to form a thin-film type. Thereafter, an NE-IQ (Pam3Cys-Lip + QS21) adjuvant was prepared using the same method as that used in Example 3.

### Example 9-4. Preparation of NE-IQ-4 Adjuvant: NE-IQ + DDA + STING (cyclic DNA)

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), c-di-AMP (2 mg, Invivo Gen, San Diego, USA), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Thereafter, an NE-IQ (DDA + STING (cyclic DNA)) adjuvant was prepared using the same method as that used in Example 3.

### Example 9-5. Preparation of NE-IQ-5 Adjuvant: NE-IQ + DDA + Poly(I:C)

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Then, the soybean oil (2.5% v/v, Sigma Aldrich, USA) including R837 prepared using the same method as that used in Example 1, except that soybean oil was used instead of squalene, and a Tween (1% v/v)-containing phosphate buffer solution (PBS, 0.0067 M PO₄) 2 mL was added, and then the lipid film was dispersed in the solution using a stirrer at 60 °C and 600 rpm for 30 minutes. The dispersion solution was transferred to a 4 mL vial, and then a tip sonicator was used for 1 minute to completely disperse the lipid film in the PBS. Lastly, Poly(I:C) (2 mg/mL, Invivo Gen, USA) was dissolved in a phosphate buffer solution (0.0067 M PO₄), and then mixed with the nanoemulsion in a volume ratio of 1:1, stirred for about 2 hours, and then stored in a refrigerator at 4 °C until use.

### Example 9-6. Preparation of NE-IQ-6 Adjuvant: NE-IQ + DDA + CL401

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Then, squalene (2.5% v/v, Sigma Aldrich, USA) including R837 prepared in the same manner as in Example 1 and a Tween (1% v/v)-containing phosphate buffer solution (PBS, 0.0067 M PO₄) 2 mL was added, and then the lipid film was dispersed in the solution using a stirrer at 60 °C and 600 rpm for 30 minutes. The dispersion solution was transferred to a 4 mL vial, and then a tip sonicator was used for 1 minute to completely disperse the lipid film in the PBS. Lastly, CL401 (2 mg/mL, Invivo Gen, San Diego, USA) was dissolved in a phosphate buffer solution (0.0067 M PO₄), and then mixed with nanoliposomes in a volume ratio of 1:1:1, stirred for about 2 hours, and then stored in a refrigerator at 4 °C until use.

### Example 9-7. Preparation of NE-IQ-7 Adjuvant: NE-IQ + DDA 7 + CL429

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Then, squalene (2.5% v/v, Sigma Aldrich, USA) including R837 prepared in the same manner as in Example 1 and a Tween (1% v/v)-containing phosphate buffer solution (PBS, 0.0067 M PO₄) 2 mL was added, and then the lipid film was dispersed in the solution using a stirrer at 60 °C and 600 rpm for 30 minutes. The dispersion solution was transferred to a 4 mL vial, and then a tip sonicator was used for 1 minute to completely disperse the lipid film in the PBS. Lastly, CL429 (2 mg, Invivo Gen, San Diego, USA) was dissolved in a phosphate buffer solution (0.0067 M PO₄), and then mixed with nanoliposomes in a volume ratio of 1:1, stirred for about 2 hours, and then stored in a refrigerator at 4 °C until use.

### Example 9-8. Preparation of NE-IQ-8 Adjuvant: NE-IQ + DDA + CpG

DOPC (1,2-dioleoyl-sn-glycero-3-phosphocholine, 1 mg/mL, Sigma Aldrich, USA), DDA (dimethyldioctadecylammonium bromide, 1 mg/mL, Sigma Aldrich, USA), and cholesterol (0.25 mg/mL, Sigma Aldrich, USA) were dissolved in 1 mL of ethanol. The solution was transferred to a round flask and the ethanol was completely evaporated using a rotary evaporator to form a thin-film type. Then, squalene (2.5% v/v, Sigma Aldrich, USA) including R837 prepared in the same manner as in Example 1 and a Tween (1% v/v)-containing phosphate buffer solution (PBS, 0.0067 M PO₄) 2 mL was added, and then the lipid film was dispersed in the solution using a stirrer at 60 °C and 600 rpm for 30 minutes. The dispersion solution was transferred to a 4 mL vial, and then a tip sonicator was used for 1 minute to completely disperse the lipid film in the PBS. Lastly, CpG (2 mg/mL, Invivo Gen, San Diego, USA) was dissolved in a phosphate buffer solution (0.0067 M PO₄), and then mixed with an nanoemulsion in a volume ratio of 1:1, stirred for about 2 hours, and then stored in a refrigerator at 4 °C until use.

### Example 10. Confirmation of Cell-Mediated T Cell Responses of NE-IQ-Based Nanoemulsions

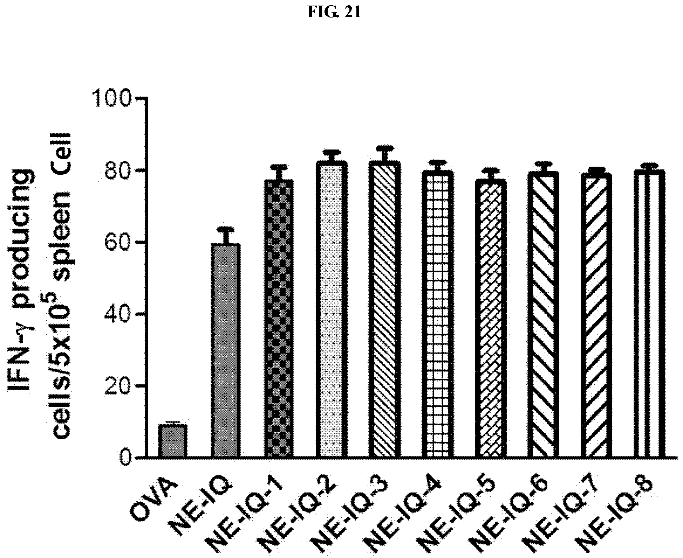

The cellular immunity of T cells for the NE-IQ-based vaccine adjuvant compositions prepared according to Examples 9-1 to 9-8, as shown in Table 2 above, was measured using the same method as that used in Example 7, and the results thereof are illustrated in FIG. 21. It was confirmed that the secretion amount of IFN-γ was dramatically increased due to the introduction of an additional immunostimulatory agent.

It will be understood by those skilled in the art that various changes in form and details may be made without departing from the essential characteristics of the invention as defined by the appended claims. Therefore, the disclosed embodiments should be considered in an illustrative rather than a restrictive sense. The scope of the present invention is defined by the appended claims rather than by the foregoing description, and all differences within the scope of equivalents thereof should be construed as within the scope of the present invention.

### [Industrial Applicability]

A nanoemulsion including an oil layer including an imidazoquinoline-based toll-like receptor 7 or 8 agonist and oil, according to the present invention, can provide a vaccine adjuvant in an emulsion form which is used in the activation of immune cells that play a vital role in vaccines for the prevention and treatment of infectious diseases and immunotherapy. In addition, an imidazoquinoline-based material is stably dispersed in oil such as squalene using a dispersion helper and the resulting oil layer is finally prepared into an emulsion form, thereby providing a vaccine adjuvant using an immidazoquinoline-based material, which is poorly soluble in most organic solvents and an aqueous solution. Meanwhile, when an adjuvant in a nanoemulsion form of the present invention is used as a vaccine after binding to an antigen, the adjuvant has an effect of significantly enhancing both humoral immunity and cellular immunity. In addition, in a nanoemulsion including an oil layer including an imidazoquinoline-based toll-like receptor 7 or 8 agonist and oil, according to the present invention, the imidazoquinoline-based toll-like receptor agonist, which is lipophilic, exhibits controlled release behavior from the oil layer, which is an inner side of the nanoemulsion, to an aqueous solution layer, and thus is slowly released *in vivo,* thereby enhancing cellular immunity and addressing systemic toxicity problems.

## Claims

1. A nanoemulsion comprising an oil layer comprising an imidazoquinoline-based toll-like receptor 7 or 8 agonist and oil.

2. The nanoemulsion of claim 1, wherein the imidazoquinoline-based toll-like receptor 7 or 8 agonist is dispersed in a nano-sized oil layer.

3. The nanoemulsion of claim 2, wherein an outer portion of the oil layer is coated with one or more selected from the group consisting of a surfactant and an immunostimulatory agent.

4. The nanoemulsion of claim 1, wherein the imidazoquinoline-based toll-like receptor 7 or 8 agonist is selected from the group consisting of imiquimod, resiquimod, dactolisib, gardiquimod, sumanirole, motolimod, and derivatives thereof.

5. The nanoemulsion of claim 1, wherein the oil is selected from the group consisting of fish-derived oil, animal-derived oil, vegetable-derived oil, a tocopherol, mineral oil, and castor oil.

6. The nanoemulsion of claim 5, wherein the animal-derived oil comprises squalene, and the vegetable-derived oil comprises oleic acid.

7. The nanoemulsion of claim 1, wherein the imidazoquinoline-based toll-like receptor 7 or 8 agonist and the oil are included in a weight ratio of 0.1:100 to 1:50.

8. The nanoemulsion of claim 1, further comprising a dispersion helper for facilitating a lipophilic interaction between the imidazoquinoline-based toll-like receptor 7 or 8 agonist and the oil.

9. The nanoemulsion of claim 8, wherein the dispersion helper is selected from the group consisting of myristoleic acid, palmitoleic acid, sapienic acid, oleic acid, elaidic acid, vaccenic acid, linoleic acid, linoelaidic acid, alpha-linoleic acid, arachidonic acid, eicosapentaenoic acid, erucic acid, docosahexaenonic acid, caprylic acid, capric acid, lauric acid, myristic acid, palmitic acid, stearic acid, arachidic acid, behenic acid, lignoceric acid, and cerotic acid.

10. The nanoemulsion of claim 8, wherein the dispersion helper and the imidazoquinoline-based toll-like receptor 7 or 8 agonist are included in a weight ratio of 0.1:10 to 10:0.1.

11. The nanoemulsion of claim 3, wherein the surfactant is selected from the group consisting of:
polyoxyethylene sorbitan ester surfactants (Tween) including polysorbate 20 and polysorbate 80;
copolymers comprising one or more selected from ethylene oxide (EO), propylene oxide (PO), and butylene oxide (BO);
octoxynols including Triton X-100 and t-octylphenoxypolyethoxyethanol; (octylphenoxy)polyethoxy ethanol;
phospholipids comprising phosphatidylcholine(lecithin) phosphatidylethanolaniline, phosphatidylserine, phosphatidylinositol, phosphatidylglycerol, phosphatidic acid, sphingomyelin, and cardiolipin;
nonylphenol etoxylates;
polyoxyethylene fatty ethers derived from lauryl, cetyl, and oleyl alcohols, including triethyleneglycol monolauryl ether; and
sorbitan esters (SPAN) comprising sorbitan trioleate (Span 85) and sorbitan monolaurate.

12. The nanoemulsion of claim 3, wherein the immunostimulatory agent is selected from the group consisting of toll-like receptor agonists (TLRs), saponins, antiviral peptides, inflammasome inducers, NOD ligands, cytosolic DNA sensor (CDS) ligands, stimulator of interferon genes (STING) ligands, and cationic lipids.

13. An adjuvant composition comprising the nanoemulsion according to any one of claims 1 to 12.

14. A vaccine composition comprising the adjuvant composition according to claim 13 and an antigen.

15. The vaccine composition of claim 14, wherein the antigen is selected from the group consisting of proteins, recombinant proteins, glycoproteins, genes, peptides, polysaccharides, lipopolysaccharides, polynucleotides, cells, and viruses.

16. A method of preparing a nanoemulsion, the method comprising:
a) preparing an oil solution by dissolving an imidazoquinoline-based toll-like receptor 7 or 8 agonist in oil;
b) mixing the oil solution with one or more selected from the group consisting of a surfactant and an immunostimulatory agent;
c) dispersing the mixed solution obtained in process b) in an aqueous solution.

17. The method of claim 16, wherein in the preparing the oil solution, a dispersion helper for facilitating a lipophilic interaction between the imidazoquinoline-based toll-like receptor 7 or 8 agonist and the oil is further dissolved in the oil.
